# EUROPEAN PATENT SPECIFICATION

(11) **EP 4 615 819 B1**
(45) Date of publication and mention of the grant of the patent: **10.06.2026**
(21) Application number: 24804541.1
(22) Date of filing: 11.11.2024
(51) Int. Cl.: C07C 67/08, C07C 69/86

(54) **PROCESS FOR THE PREPARATION OF 2-(METH)ACRYLOYLOXY BENZOIC ACID ESTERS**
VERFAHREN ZUR HERSTELLUNG VON 2-(METH)ACRYLOYLOXYBENZOESÄUREESTERN
PROCÉDÉ DE PRÉPARATION D'ESTERS D'ACIDE 2-(MÉTH)ACRYLOYLOXY BENZOÏQUE

(30) Priority: 20.11.2023 EP 23210852; 27.11.2023 EP 23212326
(43) Date of publication of application: 17.09.2025
(73) Proprietor: Evonik Operations GmbH, 45128 Essen (DE)
(72) Inventor: WALTER, Kristine, 64354 Reinheim (DE); BLEITH, Tim, 55131 Mainz (DE); SAAL, Doris, 64625 Bensheim (DE); GRÄFF, Günther, 64625 Bensheim (DE); BESTGEN, Sebastian, 97080 Würzburg (DE); SCHÜTZ, Thorben, 64665 Alsbach-Hähnlein (DE)
(74) Representative: Evonik Patent Association
(86) International application number: PCT/EP2024/081840
(87) International publication number: WO 2025/108749

(56) References cited:
- WO-A1-2019/213588

## Description

The present invention is directed to a solvent-free process for the preparation of 2-(meth)acryloyloxy benzoic acid esters wherein 2-hydroxybenzoates are reacted with (meth)acrylic acid anhydride in the presence of lithium methoxide as catalyst.

These polymerizable monomers can be used in a variety of applications, including the use as reactive diluent in photopolymerization processes, such as lithography-based photopolymerization processes, for example high temperature lithography-based processes conducted at temperatures greater than 90°C. The monomers allow for good processibility in additive manufacturing techniques and may yield cured products having thermomechanical properties that are desirable for various applications, including for the formation of medical devices and/or those items used in an intraoral environment, e.g., intraoral devices, such as aligners, expanders or spacers.

### State of the art

WO 2019/213588 is directed to photopolymerizable monomers and discloses the preparation of certain 2-(meth)acryloyloxy benzoic acid esters by reacting 2-hydroxy benzoates with (meth)acryloyl chloride.

The use of (meth)acrylic anhydride as reagent is described for the substrate menthyl salicylate in the presence of DMAP as catalyst without the use of a solvent (see Example 3, [0185]).

A solvent-free preparation of 2-(meth)acryloyloxy benzoic acid esters in the presence of lithium methoxide as catalyst is not described.

Liska et. al. (Journal of Polymer Science 2021 (Hoboken, NJ, United States), Volume 59, Issue 19, Pages 2154-2169) relates to a method for producing salicylate methacrylates in dichloromethane using stochiometric amounts of triethyl amine and methacryloyl chloride. The precipitated ammonium chloride was removed by filtration. The filtrate was washed, and the organic layer was dried over Na₂SO₄, filtered and stabilized, Subsequently, the solvent was evaporated. The crude product was purified by silica chromatography.

A solvent-free preparation of 2-(meth)acryloyloxy benzoic acid esters in the presence of lithium methoxide as catalyst is not described.

WO 2019/224193 relates to a method for preparing keto-functionalized aromatic (meth)acrylates by reacting keto-functionalized aromatic alcohols or keto-functionalized aromatic amines with (meth)acrylic anhydride having a content of (meth)acrylic acid anhydride of less than 4.5%. Exemplary products are benzophenone derivatives, prepared in the presence of catalytic amounts of concentrated sulfuric acid or aqueous sodium hydroxide solution.

The solvent-free preparation of 2-(meth)acryloyloxy benzoic acid esters in the presence of lithium methoxide as catalyst is not described.

WO 2020/035315 relates to a method for preparing (meth)acrylic acid esters of primary, secondary and tertiary alcohols as well as phenols using (meth)acrylic anhydride in the presence of a magnesium or rare earth element.

The preparation of 2-(meth)acryloyloxy benzoic acid esters in the presence of lithium methoxide as catalyst is not described.

JP 2022-147037 relates to a method for reacting 2-hydroxy-4'-(2-hydroxyethoxy)-2-methylpropiophenone with (meth)acrylic anhydride in the presence of a lithium, magnesium or calcium salt. The preparation of 2-(meth)acryloyloxy benzoic acid esters in the presence of lithium methoxide as catalyst is not described.

JP 2022-147036 relates to a method for producing benzophenone methacrylate with (meth)acrylic anhydride in the presence of a lithium, magnesium or calcium salt. The preparation of 2-(meth)acryloyloxy benzoic acid esters in the presence of lithium methoxide as catalyst is not described.

It was an object of the present invention to provide a solvent-free method for preparing 2-(meth)acryloyloxy benzoic acid esters in high yield and purity which can be used in large scales and avoids the preparation of equimolar amounts of solid by-products which have to be removed by intensive work-up to isolate the desired product.

It was surprisingly found that the sterically hindered alcohols 2-hydroxybenzoates can be converted to the respective 2-(meth)acryloyloxy benzoic acid esters using (meth)acrylic anhydride as the reagent in the presence of lithium methoxide as catalyst.

Due to the steric hinderance of the alcohol, the commonly known equimolar reactions using known catalysts as described above are not feasible.

### Detailed description of the invention

The present invention is directed to a process for preparing 2-(meth)acryloyloxy benzoic acid esters of general formula (I) 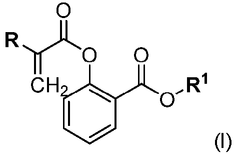 wherein
**R** denotes H or CH₃ and
**R¹** denotes a C5-7 cycloalkyl group which is unsubstituted or substituted with one, two or three substituents selected from the group consisting of straight-chained or branched C1-6 alkyl, straight-chained or branched C1-6 alkyloxy, -O(CO)-(C1-6)alkyl, -COO-(C1-6)alkyl, or
**R¹** denotes a residue of general formula (II) 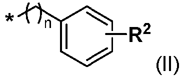 wherein
**R²** denotes a straight-chained or branched C1-6 alkyl group or a straight-chained or branched C1-6 alkyloxy group and
**n** denotes an integer 0, 1 or 2,
the process comprising the step of:
(a) reacting a 2-hydroxybenzoate of general formula (III): 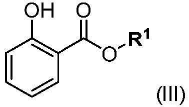 wherein
   **R¹** is defined as disclosed herein before,
   with (meth)acrylic acid anhydride of general formula (IV) 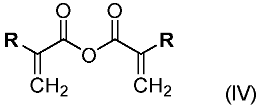
   wherein **R** denotes H or CH₃,
   in the presence of lithium methoxide as catalyst and at least one stabilizer,
   characterized in that the reaction takes place in the absence of any solvent.

A further object of the present invention is directed to a process for preparing 2-(meth)acryloyloxy benzoic acid esters of general formula (I), wherein **R** denotes H or CH₃ and **R¹** denotes a cyclohexyl group which is unsubstituted or substituted with one, two or three substituents selected from the group consisting of straight-chained or branched C1-3 alkyl and straight-chained or branched C1-3 alkyloxy, or a benzyl group.

A further embodiment is directed to the process as outlined further above, wherein the 2-(meth)acryloyloxy benzoic acid ester is 3,3,5-trimethylcyclohexyl salicyl (meth)acrylate of general formula (la): 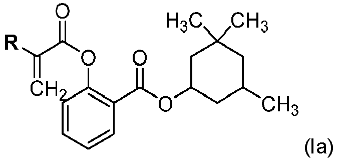 wherein **R** denotes H or CH₃,
the process comprising the step of:
(a) reacting 3,3,5-trimethylcyclohexyl-2-hydroxybenzoate of formula (Illa): 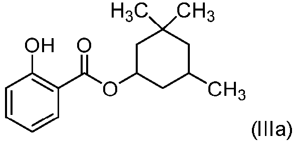
   with (meth)acrylic acid anhydride of general formula (IV) 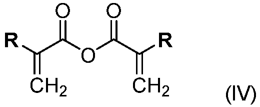
   wherein **R** denotes H or CH₃,
   in the presence of lithium methoxide as catalyst and at least one stabilizer,
   characterized in that the reaction takes place in the absence of any solvent.

The term "(meth)acrylate" refers to both, esters of acrylic acid and esters of methacrylic acid.

The term "(meth)acrylic acid anhydride" refers to both, acrylic acid anhydride and methacrylic acid anhydride.

The term "(meth)acrylic acid" refers to both, acrylic acid and methacrylic acid.

The C5-7 cycloalkyl group for use in accordance with the present invention can be selected from the group consisting of cyclopentyl, cyclohexyl and cycloheptyl.

The straight-chained or branched C1-6 alkyl group for use in accordance with the present invention can be selected from the group consisting of methyl, ethyl, propyl, isopropyl, butyl, isobutyl, tert-butyl, pentyl and hexyl.

The straight-chained or branched C1-3 alkyl group for use in accordance with the present invention can be selected from the group consisting of methyl, ethyl, propyl and isopropyl.

The straight-chained or branched C1-6 alkyloxy group for use in accordance with the present invention can be selected from the group consisting of methyloxy, ethyloxy, propyloxy, isopropyloxy, butyloxy, isobutyloxy, tert-butyloxy, pentyloxy and hexyloxy.

The straight-chained or branched C1-3 alkyloxy group for use in accordance with the present invention can be selected from the group consisting of methyloxy, ethyloxy, propyloxy and isopropyloxy.

In accordance with the invention, the catalyst lithium methoxide is used in an amount of 3.5 mol% to 10 mol%, preferably 3.5 mol% to 7 mol%, based on the amount of the 2-hydroxybenzoate of general formula (III) or (IIIa) used as starting material.

Suitable stabilizers are known to those skilled in the art. They include, for example, phenothiazine, substances having an oxyl radical, such as 2,2,6,6-tetramethylpiperidinyl-N-oxyl (TEMPO), 4-hydroxy-2,2,6,6-tetramethylpiperidinyl-N-oxyl (TEMPOL) or 4-(meth)acryloyloxy-2,2,6,6-tetramethylpiperidinyl-N-oxyl (TEMPOL-(meth)acrylate) and also phenol derivatives such as hydroquinone monomethyl ether (HQME), 2,4-dimethyl-6-tert-butylphenol (DMBP), 2,6-di-tert-butylphenol or 4-methyl-2,6-di-tert-butylphenol (BHT). Mixtures of different stabilizers can also be used. Preference is given to using HQME, DMBP, BHT, phenothiazine and also mixtures of these substances.

The applications of 2-(meth)acryloyloxy benzoic acid esters (I) and especially 3,3,5-trimethylcyclohexyl salicyl (meth)acrylate (Ia) generally require colourless products. Therefore, for these unsaturated compounds, preference is given to using non-colouring stabilizers or colouring stabilizers in very small amounts. The used amount of stabilizer is dependent on the starting materials.

The amount of stabilizer at the start of the reaction is adjusted to be between 1 ppm and 5000 ppm, based on the amount of the 2-hydroxybenzoate of general formula (III) or (Illa) used as starting material. Preferably, the amount of stabilizer at the start of the reaction is adjusted to be between 1 ppm and 3000 ppm, particularly preferably between 1 ppm and 2000 ppm, based on the amount of the 2-hydroxybenzoate of general formula (III) or (Illa) used as starting material.

The reaction is carried out in a suitable reaction vessel that is equipped with a stirrer and a temperature control system under air conditions.

The reaction may be carried out at standard pressure or elevated pressure.

The reaction temperature is generally in the range of 95°C to 115°C, preferably 100°C to 110°C.

The reaction is usually run until all starting material is reacted (GC control).

In accordance with the present invention, reaction times of 4 hours up to 15 hours are preferred.

In order to completely transfer the sterically hindered alcohol to the corresponding product, the use of an excess of (meth)acrylic acid anhydride is necessary.

In accordance with the present invention, 1.5 to 2.5 mol equivalents, preferably about 2 mol equivalents of (meth)acrylic acid anhydride are used, based on the amount of the 2-hydroxybenzoate of general formula (III) or (Illa) used as starting material.

During the reaction, (meth)acrylic acid is formed as a by-product. Consequently, the reaction mixture retrieved in step (a) contains not only the desired product of general formula (I) or (la), but also (meth)acrylic acid as well as the excessive (meth)acrylic acid anhydride.

The presence of the catalyst leads to deposits and polymerization during distillation. Consequently, the catalyst must be removed before further purification of the crude product can be done.

To remove the catalyst from the product retrieved in step (a) as outlined further above, the process mixture retrieved in step (a) can be diluted with water in a ratio of 1:10 to 10:1, preferably of 1:1 to 10:1 and stirred for at least 10 minutes.

A further object of the present invention is therefore directed to the process comprising step (a) as outlined further above and also comprising the following step (b):
(b) mixing the process mixture retrieved in step (a) with water in a ratio of 1:10 to 10:1, preferably of 1:1 to 10:1 and stirring the resulting mixture for at least 10 minutes.

To separate the aqueous phase of the mixture retrieved in step (b) from the product phase, a phase separation can be carried out. This phase separation can be done by commonly known methods, for example by using simple extraction methods or methods based on gravity like centrifuge, separator or coalescer. In accordance with the present invention, the preferred separation method is by using a centrifuge.

A further object of the present invention is therefore directed to the process comprising steps (a) and (b) as outlined further above and also comprising the following step (c):
(c) separating the aqueous phase of the mixture retrieved in step (b) from the product phase to remove the catalyst.

The product retrieved in step (c) may still contain undesired impurities. To especially remove low-boiling by-products like (meth)acrylic acid and (meth)acrylic acid anhydride, the product retrieved in step (c) can undergo a first distillation to retrieve the product with a content of (meth)acrylic acid and (meth)acrylic acid anhydride of below 1%.

If the content of the undesired by-products is still too high, this distillation can be repeated for at least a second time.

The distillation may be carried out under ambient pressure or reduced pressure and at temperatures in the range of 20°C to 150°C, preferably 70°C to 150°C.

A further object of the present invention is therefore directed to the process comprising steps (a), (b) and (c) as outlined further above and also comprising the following step (d):
(d) distillation of the product phase retrieved in step (c) to retrieve the desired product with a content of (meth)acrylic acid and (meth)acrylic acid anhydride of below 1% (distillation may have to be repeated at least one further time).

To further purify the product retrieved in step (d), especially for removing high-boiling impurities, the product can undergo a second distillation process to retrieve the desired product with a purity of 90% or higher.

The distillation may be carried out under reduced pressure and at temperatures in the range of 100°C to 200°C, preferably 120°C to 180°C.

A further object of the present invention is therefore directed to the process comprising steps (a), (b), (c) and (d) as outlined further above and also comprising the following step (e):
(e) optionally, subsequent additional distillation of the product retrieved in step (d) to separate from further impurities (high boiling compounds).

A further object of the present invention is directed to a process for preparing 3,3,5-trimethylcyclohexyl salicyl (meth)acrylate of general formula (la): 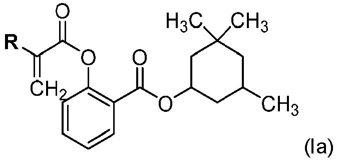 wherein R denotes H or CH₃,
comprising the steps of:
(a) reacting 3,3,5-trimethylcyclohexyl-2-hydroxybenzoate of formula (Illa): 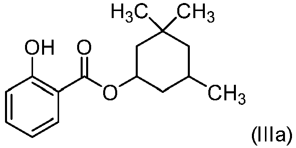
   with (meth)acrylic acid anhydride of general formula (IV) 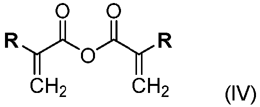
   wherein R denotes H or CH₃,
   in the presence of lithium methoxide as catalyst and at least one stabilizer,
   characterized in that the reaction takes place in the absence of any solvent;
(b) mixing the process mixture retrieved in step (a) with water in a ratio of 1:10 to 10:1, preferably of 1:1 to 10:1 and stirring the resulting mixture for at least 10 minutes;
(c) separating the aqueous phase of the mixture retrieved in step (b) from the product phase to remove the catalyst;
(d) distillation of the product phase retrieved in step (c) to retrieve the desired product with a content of (meth)acrylic acid and (meth)acrylic acid anhydride of below 1%; and
(e) optionally, subsequent additional distillation of the product retrieved in step (d) to separate it from further impurities.

The present invention is intended to be described in more detail below using the examples and comparative examples, without this giving rise to any restriction.

### Experimental Part

### Abbreviations

- CE: comparative example
- DMAP: 4-dimethylaminopyridine
- HOMOSALATE: 3,3,5-trimethylcyclohexyl-2-hydroxybenzoate
- HSMA: HOMOSALATE methacrylate = 3,3,5-trimethylcyclohexyl salicyl methacrylate
- MA: methacrylic acid
- MAAH: methacrylic acid anhydride
- Me: methyl

### Analytical Measurements

### Determination of the process mixture composition by gas chromatography (GC):

To determine the process mixture composition, a sample of the composition in acetone (20% solution) was investigated by gas chromatographic analysis. For this purpose, an "Agilent 7820" instrument from Agilent was used with a column of the DB5 type (length: 30.0 m, diameter: 250.00 µm, film thickness: 0.25 µm). The injection amount was 0.2 µL (split 1:60). The instrument was operated at an injector temperature of 300°C and a detector temperature of 300°C. The following temperature program was used: 80°C for 2 minutes, heating to 300°C at a heating rate of 16°C per minute and maintaining the final temperature for further 6 minutes.

### Determination of color number:

The color number was determined by the means of the process explained in detail in US 2004/186311 (determination of color by the platinum-cobalt scale; also called APHA number). This process was developed based on DIN EN ISO 6271. In accordance with the present invention, a Cary 100 UV/VIS spectrometer from Agilent Technologies with filters for the range of 460 and 620 nm was used.

### Examples

### Preparation of a stock solution for the preparation of examples 3 and 4, comparative examples 1-4, 10-29 and 31

### targeted molar ratio of homosalate : MAAH = 1:2

78.7 g of homosalate (0.3 mol) and 92.5 g of MAAH (0.6 mol) were combined with 17.2 mg of phenothiazine (100 ppm) and stirred to obtain a stock solution with a molar ratio of homosalate : MAAH of 1:2.

### Preparation of a stock solution for the preparation of examples 1 and 2, comparative examples 5-9 targeted molar ratio of homosalate : MAAH = 1:2.12

26.2 g of homosalate (0.1 mol) and 32.7 g of MAAH (0.212 mol) were combined with 5.2 mg of phenothiazine (100 ppm) and stirred to obtain a stock solution with a molar ratio of homosalate : MAAH of 1:2.12.

### Preparation of a stock solution for the preparation of comparative examples 30, 32 and 33 targeted molar ratio of homosalate : MAAH = 1:1.7

26.2 g of homosalate (0.1 mol) and 26.2 g of MAAH (0.17 mol) were combined with 5.2 mg of phenothiazine (100 ppm) and stirred to obtain a stock solution with a molar ratio of homosalate : MAAH of 1:1.7.

### General procedure for the preparation of examples 3 and 4 and comparative examples 1-4 and 7-33

7.0 g samples of the corresponding stock solution were placed in a 15 mL pressure tube with a Teflon^{®} plug and a magnetic agitator. To this solution, the catalyst was added as indicated in Table 1 (mol% with respect to the amount of homosalate). The pressure tube was tightly closed. Subsequently, the pressure tube was placed in an aluminum heating block with an integrated magnetic stirrer and stirred. The respective time and temperature are outlined in Table 1. Samples for GC analysis were filtered through a syringe filter unit and diluted with acetone (20% solution) prior to analysis.

### General procedure for the preparation of comparative examples 5 and 6 with 1.5 mol% catalyst

A four-necked 2 liter round bottom flask was equipped with a mechanical stirrer, a thermometer and a reflux condenser and connected to an air inlet. 262.4 g of homosalate (1.0 mol), 326.8 g of methacrylic anhydride (2.12 mol) (molar ratio of homosalate : MAAH of 1:2.12) and 569.7 mg of lithium methoxide (15.0 mmol) were added and the mixture was stabilized with 59 mg of phenotiazine (100 ppm). The reaction mixture was heated to 100°C and samples were taken after 4 and 8 hours. Samples for GC analysis were filtered through a syringe filter unit and diluted with acetone (20% solution) prior to analysis.

### General procedure for the preparation of examples 1 and 2 with 3.5 mol% catalyst

A four-necked 2 liter round bottom flask was equipped with a mechanical stirrer, a thermometer and a reflux condenser and connected to an air inlet. 262.3 g of homosalate (1.0 mol), 326.8 g of methacrylic anhydride (2.12 mol) (molar ratio of homosalate : MAAH of 1:2.12) and 1.3 g of lithium methoxide (35.4 mmol) were added were added and the mixture was stabilized with 59 mg of phenotiazine (100 ppm). The reaction mixture was heated to 100°C and samples were taken after 4 and 8 hours. Samples for GC analysis were filtered through a syringe filter unit and diluted with acetone (20% solution) prior to analysis.

**Table 1: Reaction conditions**

| Example | Catalyst | Catalyst amount | Catalyst amount | Reaction time | **Reaction** temperature |
|---|---|---|---|---|---|
| # | | [g] | [mol%] | [h] | [°C] |
| CE 1 | LiOMe | 0.0023 | 0.5 | 4 | 90 |
| CE 2 | LiOMe | 0.0023 | 0.5 | 8 | 90 |
| CE 3 | LiOMe | 0.0093 | 2 | 4 | 90 |
| CE 4 | LiOMe | 0.0093 | 2 | 8 | 90 |
| CE 5 | LiOMe | See procedure | 1.5 | 4 | 100 |
| CE 6 | LiOMe | See procedure | 1.5 | 8 | 100 |
| Example 1 | LiOMe | See procedure | 3.5 | 4 | 100 |
| Example 2 | LiOMe | See procedure | 3.5 | 8 | 100 |
| Example 3 | LiOMe | 0.0273 | 6 | 4 | 100 |
| Example 4 | LiOMe | 0.0273 | 6 | 8 | 100 |
| CE 7 | LiOMe | 0.0267 | 6 | 4 | 120 |
| CE 8 | LiOMe | 0.0267 | 6 | 8 | 120 |
| CE 9 | LiOMe | 0.0267 | 6 | 24 | 120 |
| CE 10 | NaOMe | 0.0399 | 6 | 4 | 90 |
| CE 11 | NaOMe | 0.0399 | 6 | 8 | 90 |
| CE 12 | NaOMe | 0.0399 | 6 | 4 | 100 |
| CE 13 | NaOMe | 0.0399 | 6 | 8 | 100 |
| CE 14 | KOMe | 0.0528 | 6 | 4 | 90 |
| CE 15 | KOMe | 0.0528 | 6 | 8 | 90 |
| CE 16 | KOMe | 0.0528 | 6 | 4 | 100 |
| CE 17 | KOMe | 0.0528 | 6 | 8 | 100 |
| CE 18 | MpCl₂ | 0.0703 | 6 | 4 | 90 |
| CE 19 | MpCl₂ | 0.0703 | 6 | 8 | 90 |
| CE 20 | MpCl₂ | 0.0703 | 6 | 4 | 100 |
| CE 21 | MgBr₂ | 0.1358 | 6 | 4 | 90 |
| CE 22 | MgO | 0.0494 | 10 | 4 | 65 |
| CE 23 | MgO | 0.0297 | 6 | 4 | 90 |
| CE 24 | MgO | 0.0297 | 6 | 4 | 100 |
| CE 25 | Mg salt of MA | 0.2391 | 10 | 4 | 65 |
| CE 26 | Mg salt of MA | 0.1435 | 6 | 4 | 90 |
| CE 27 | Mg salt of MA | 0.1435 | 6 | 8 | 90 |
| CE 28 | Mg salt of MA | 0.1435 | 6 | 4 | 100 |
| CE 29 | DMAP | 0.073 | 5 | 4 | 100 |
| CE 30 | DMAP | 0.0806 | 5 | 4 | 120 |
| CE 31 | DMAP | 0.073 | 5 | 8 | 100 |
| CE 32 | DMAP | 0.0806 | 5 | 8 | 120 |
| CE 33 | DMAP | 0.0806 | 5 | 24 | 120 |

Examples 1-4 are in accordance with the present invention and disclose the preparation of 3,3,5-trimethylcyclohexyl salicyl (meth)acrylate from 3,3,5-trimethylcyclohexyl-2-hydroxybenzoate by using lithium methoxide as catalyst.

Comparative examples 1-33 use different catalysts for the same reaction.

**Table 2: Residual homosalate, homosalate methacrylate (HSMA) conversion and high boiler impurities determined by gas chromatography (in area %)**

| # | Catalyst | Time | Temperature | Homosalate | HSMA | High boilers | HSMA : Homosalate |
|---|---|---|---|---|---|---|---|
| | [mol%] | [h] | [°C] | [area %] | [area %] | [area %] | |
| CE 1 | LiOMe (0.5) | 4 | 90 | 28.1 | 30.7 | 0.4 | 1.1 |
| CE 2 | LiOMe (0.5) | 8 | 90 | 21.8 | 38.3 | 0.8 | 1.8 |
| CE 3 | LiOMe (2) | 4 | 90 | 20.3 | 39.8 | 0.9 | 2.0 |
| CE 4 | LiOMe (2) | 8 | 90 | 15.1 | 46.2 | 1.4 | 3.1 |
| CE 5 | LiOMe (1.5) | 4 | 100 | 11.0 | 48.4 | 1.6 | 4.4 |
| CE 6 | LiOMe (1.5) | 8 | 100 | 5.0 | 58.0 | 2.1 | 11.6 |
| Example 1 | LiOMe (3.5) | 4 | 100 | 4.1 | 56.6 | 2.3 | 13.9 |
| Example 2 | LiOMe (3.5) | 8 | 100 | 1.5 | 59.2 | 2.8 | 38.4 |
| Example 3 | LiOMe (6) | 4 | 100 | 3.0 | 59.0 | 2.7 | 20.0 |
| Example 4 | LiOMe (6) | 8 | 100 | 1.3 | 61.6 | 3.0 | 48.2 |
| CE 7 | LiOMe (6) | 4 | 120 | 1.9 | 64.9 | 3.5 | 34.6 |
| CE 8 | LiOMe (6) | 8 | 120 | 1.8 | 64.4 | 4.1 | 36.6 |
| CE 9 | LiOMe (6) | 24 | 120 | 2.3 | 63.4 | 6.8 | 27.8 |
| CE 10 | NaOMe (6) | 4 | 90 | 10.3 | 50.8 | 3.1 | 4.9 |
| CE 11 | NaOMe (6) | 8 | 90 | 4.0 | 57.6 | 3.9 | 14.5 |
| CE 12 | NaOMe (6) | 4 | 100 | 11.6 | 49.3 | 2.8 | 4.3 |
| CE 13 | NaOMe (6) | 8 | 100 | 3.6 | 58.5 | 4.0 | 16.4 |
| CE 14 | KOMe (6) | 4 | 90 | 30.3 | 27.7 | 2.0 | 0.9 |
| CE 15 | KOMe (6) | 8 | 90 | 23.9 | 34.7 | 2.9 | 1.5 |
| CE 16 | KOMe (6) | 4 | 100 | 25.9 | 32.3 | 2.1 | 1.2 |
| CE 17 | KOMe (6) | 8 | 100 | 12.8 | 40.7 | 3.5 | 3.2 |
| CE 18 | MpCl₂ (6) | 4 | 90 | 12.2 | 57.4 | 1.9 | 4.7 |
| CE 19 | MpCl₂ (6) | 8 | 90 | 5.1 | 65.4 | 2.3 | 12.9 |
| CE 20 | MpCl₂ (6) | 4 | 100 | 5.6 | 57.0 | 1.5 | 10.3 |
| CE¹ 21 | MaBr₂ (6) | 4 | 90 | 3.4 | 58.6 | 1.6 | 17.4 |
| CE 22 | MgO (10) | 4 | 65 | 32.1 | 25.6 | 0.3 | 0.8 |
| CE 23 | MgO (6) | 4 | 90 | 7.4 | 56.0 | 1.5 | 7.6 |
| CE 24 | MgO (6) | 4 | 100 | 4.1 | 59.3 | 1.6 | 14.3 |
| CE 25 | Mg salt of MA (10) | 4 | 65 | 30.9 | 26.2 | 0.6 | 0.8 |
| CE 26 | Mg salt of MA (6) | 4 | 90 | 9.7 | 52.1 | 1.9 | 5.4 |
| CE 27 | Mg salt of MA (6) | 8 | 90 | 3.1 | 59.6 | 2.7 | 19.5 |
| CE 28 | Mg salt of MA (6) | 4 | 100 | 5.3 | 56.9 | 1.8 | 10.7 |
| CE¹ 29 | DMAP (5) | 4 | 100 | 18.1 | 37.2 | 4.0 | 2.1 |
| CE¹ 30 | DMAP (5) | 4 | 120 | 3.1 | 54.8 | 5.6 | 17.8 |
| CE¹ 31 | DMAP (5) | 8 | 100 | 8.0 | 49.1 | 6.6 | 6.2 |
| CE¹ 32 | DMAP (5) | 8 | 120 | 3.0 | 54.1 | 5.9 | 18.3 |
| CE¹ 33 | DMAP (5) | 24 | 120 | 2.8 | 57.6 | 8.0 | 20.9 |

| | | | | | | | |
|---|---|---|---|---|---|---|---|
| ¹Brown process mixture | | | | | | | |

### Conclusions:

Table 2 shows that only the use of lithium methoxide in amounts of at least 3.5 mol%, reaction temperatures of more than 90°C and less than 120°C and reaction times of 4-8 hours lead to the desired product with acceptable yields, less than 3 wt% of homosalate starting material and 3 wt% or less of undesired high boiling by-products.

### Pilot scale production trial:

The following amounts of raw materials were subsequently charged into a 1m³ reactor: 195 kg of homosalate (0.74 kmol), 243 kg of MAAH (1.58 kmol, molar ratio of homosalate : MAAH of 1:2.12), 1 kg of lithium methoxide (3.5 mol%) and 48 g of phenothiazine (110 ppm). The reaction mixture was heated to 100°C for 12 hours. The reaction progress was monitored with GC analysis. Samples were taken after 2, 4, 8 and 12 hours. Corresponding data are reported in the following Table 3.

**Table 3: Reaction progress of pilot scale production trial: Residual homosalate, homosalate methacrylate (HSMA) conversion and high boiler impurities determined by gas chromatography (in area %)**

| Time [h] | Homosalate [area %] | HSMA [area %] | High boilers [area %] | HSMA : Homosalate |
|---|---|---|---|---|
| 2 | 6.6 | 54.2 | 1.5 | 8.2 |
| 4 | 3.5 | 57.1 | 2.0 | 16.4 |
| 8 | 1.6 | 59.7 | 2.5 | 38.0 |
| 12 | 1.5 | 60.4 | 2.7 | 40.0 |

After a reaction time of 12 hours, the resulting process mixture comprising the catalyst was cooled down to room temperature.

Subsequently, storage tests of the process mixture comprising the catalyst were run at 30°C and 50°C. The results are reported in the following Tables 4a and 4b.

**Table 4a: Storage tests of the process mixture comprising the catalyst at 30°C**

| | Start | 1 month | 3 months | 6 months |
|---|---|---|---|---|
| HSMA content [area %] | 59.51 | 59.88 | 58.31 | 59.91 |
| Reactive ester content [area %] | 94.36 | 94.77 | 94.26 | 93.59 |
| MAAH content [area %] | 22.3 | 21.89 | 22.40 | 20.59 |
| MA content [area %] | 9.18 | 9.06 | 9.25 | 8.16 |
| Color [APHA] | 13 | 13 | 14 | 14 |
| Phenothiazine [ppm] | 71 | 62 | 57 | 52 |

**Table 4a continued**

| | Start | 8 months | 10 months | 12 months |
|---|---|---|---|---|
| HSMA content [area %] | 59.51 | 60.01 | 56.25 | 59.02 |
| Reactive ester content [area %] | 94.36 | 93.61 | 89.22 | 91.28 |
| MAAH content [area %] | 22.3 | 20.13 | 19.81 | 19.37 |
| MA content [area %] | 9.18 | 8.04 | 8.18 | 7.25 |
| Color [APHA] | 13 | 15 | 15 | 15 |
| Phenothiazine [ppm] | 71 | 54 | 44 | 39 |

**Table 4b: Storage tests of the process mixture comprising the catalyst at 50°C**

| | Start | 1 month | 3 months | 6 months |
|---|---|---|---|---|
| HSMA content [area %] | 59.51 | 60.03 | 57.70 | 58.19 |
| Reactive ester content [area %] | 94.36 | 93.31 | 89.81 | 84.91 |
| MAAH content [area %] | 22.3 | 20.77 | 20.33 | 17.03 |
| MA content [area %] | 9.18 | 8.27 | 7.57 | 5.33 |
| Color [APHA] | 13 | 16 | 18 | 24 |
| Phenothiazine [ppm] | 71 | 52 | 39 | 35 |

**Table 4b continued**

| | Start | 8 months | 10 months | 12 months |
|---|---|---|---|---|
| HSMA content | 59.51 | 58.53 | 56.51 | 54.74 |

| [area %] | | | | |
|---|---|---|---|---|
| Reactive ester content [area %] | 94.36 | 83.50 | 79.30 | 76.45 |
| MAAH content [area %] | 22.3 | 15.65 | 14.43 | 13.91 |
| MA content [area %] | 9.18 | 4.41 | 3.64 | 2.90 |
| Color [APHA] | 13 | 27 | 30 | 35 |
| Phenothiazine [ppm] | 71 | 32 | 24 | 20 |

### Conclusions:

Table 4a shows that the process mixture comprising the catalyst remains stable over a period of up to 12 months when stored at 30°C; i.e. the degree of purity remains almost the same within the scope of measurement accuracy. In addition, the color number of the process mixture that still comprises the catalyst only raised from 13 APHA to 15 APHA.

Table 4b shows that the process mixture comprising the catalyst remains stable over a period of at least 6 months when stored at 50°C; i.e. the degree of purity remains almost the same within the scope of measurement accuracy. In addition, the color number of the process mixture that still comprises the catalyst only raised from 13 APHA to 27 APHA.

After a storage time of 6 months, the purity slightly dropped, together with the content of the stabilizer phenothiazine. This is visible in a raise of the color number as well.

### Extraction of HSMA process mixture:

The HSMA process mixture obtained from the pilot trial was mixed continuously with water (ratio of 10 parts process mixture to 3 parts distilled water) using a reactor equipped with a stirrer (200 rpm). The residence time was 15 minutes. For the phase separation, a centrifuge V02 from CINC was used. The centrifuge was operated using the following parameters: 25°C process temperature, no mixing adapter, 21.59 mm weir, 4800 rpm, feed rate of 15 kg/hour.

433 kg of process mixture were extracted to yield 429 kg of extracted process mixture. The extracted process mixture was obtained with a residual water content of 0.0125 wt% and a residual lithium content of 9 µg/g. The extracted process mixture did not any more contain the catalyst.

### Distillation of HSMA process mixture:

For the distillation of the extracted HSMA process mixture, a thin film evaporator (length: 300 mm, diameter: 25 mm) was equipped with a condenser to collect the distillate, a Thiele-Anschütz adapter and a stirrer (IKA RE162 at 450 rpm). The vacuum was established using a rotary vane pump TRIVAC - D 25 B from Leybold and controlled with a CVC 3000 and VAP5 controller from Vacuubrand. The temperature was controlled using a circulation thermostat operated with oil as fluid.

### First distillation to remove light boilers:

1295.6 g of the extracted HSMA process mixture was fed into the thin film evaporator over a period of 98 minutes using a jacket temperature of 130°C and a vacuum of 0.4 mbar. 525.1 g of distillate were removed, and a residue of 759.3 g was obtained.

### Second distillation to remove light boilers:

743.4 g of the residue of the first pass distillation was fed again into the thin film evaporator over a period of 47 minutes using a jacket temperature of 130°C and a vacuum of 0.8 mbar. 3.6 g of distillate were removed, and a residue of 735.8 g was obtained.

### Final distillation: product distillation

721.6 g of the residue of the second pass distillation was fed again into the thin film evaporator over a period of 141 minutes using a jacket temperature of 180°C and a vacuum of 0.5 mbar. 476.2 g of final HSMA product (38% distillation yield) were obtained and 242.1 g of residue were removed.

**Table 5: Gas chromatography analysis of the final HSMA product:**

| Methacrylic acid [area %] | MAAH [area %] | Homosalate [area %] | HSMA [area %] | High boilers [area %] |
|---|---|---|---|---|
| 0.7 | 0.2 | 2.0 | 91.4 | 1.4 |

Subsequently, storage tests were run with the distilled final HSMA product at 30°C and 50°C. The results are reported in the following Tables 6a and 6b.

**Table 6a: Storage tests with the final HSMA product at 30°C**

| | Start | 1 month | 3 months | 6 months |
|---|---|---|---|---|
| HSMA content [area %] | 92.64 | 93.12 | 92.58 | 93.00 |
| Phenothiazine [ppm] | 95 | 107 | 107 | 94 |
| Color [APHA] | 9 | 9 | 9 | 10 |

**Table 6a continued**

| | Start | 8 months | 10 months | 12 months |
|---|---|---|---|---|
| HSMA content [area %] | 92.64 | 93.40 | 91.76 | 92.24 |
| Phenothiazine [ppm] | 95 | 95 | 94 | 94 |
| Color [APHA] | 9 | 14 | 16 | 21 |

**Table 6b: Storage tests with the final HSMA product at 50°C**

| | Start | 1 month | 3 months | 6 months |
|---|---|---|---|---|
| HSMA content [area %] | 92.64 | 93.12 | 92.26 | 92.67 |
| Phenothiazine [ppm] | 95* | 100 | 95 | 77 |
| Color [APHA] | 9 | 12 | 24 | 38 |

| | | | | |
|---|---|---|---|---|
| * measurement error ±5% | | | | |

**Table 6b continued**

| | Start | 8 months | 10 months | 12 months |
|---|---|---|---|---|
| HSMA content [area %] | 92.64 | 92.79 | 92.53 | 92.79 |
| Phenothiazine [ppm] | 95 | 74 | 67 | 67 |
| Color [APHA] | 9 | 57 | 68 | 80 |

### Conclusions:

Table 6a shows that the final HSMA product remains stable over a period of up to 12 months when stored at 30°C; i.e. the degree of purity remains almost the same within the scope of measurement accuracy. In addition, the color number of the final HSMA product only raised slightly from 9 APHA to 21 APHA.

Table 6b shows that the final HSMA product remains stable over a period of at least 6 months when stored at 50°C; i.e. the degree of purity remains almost the same within the scope of measurement accuracy. In addition, the color number of the final HSMA product that still comprises the catalyst only raised from 9 APHA to 38 APHA.

After a storage time of 6 months, the purity slightly dropped, together with the content of the stabilizer phenothiazine. This is visible in a raise of the color number as well.

## Claims

1. Process for preparing 2-(meth)acryloyloxy benzoic acid esters of general formula (I) 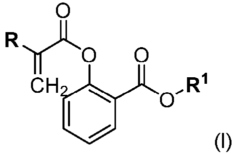 wherein
R denotes H or CH₃ and
**R¹** denotes a C5-7 cycloalkyl group which is unsubstituted or substituted with one, two or three substituents selected from the group consisting of straight-chained or branched C1-6 alkyl, straight-chained or branched C1-6 alkyloxy, -O(CO)-(C1-6)alkyl, -COO-(C1-6)alkyl, or
**R¹** denotes a residue of general formula (II) 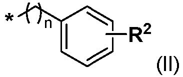
wherein
R² denotes a straight-chained or branched C1-6 alkyl group or a straight-chained or branched C1-6 alkyloxy group and
n denotes an integer 0, 1 or 2,
the process comprising the step of:
(a) reacting a 2-hydroxybenzoate of general formula (III): 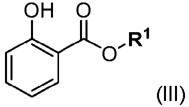 wherein
R¹ is defined as disclosed herein before,
with (meth)acrylic acid anhydride of general formula (IV) 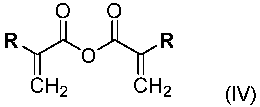
wherein R denotes H or CH₃,
in the presence of lithium methoxide as catalyst and at least one stabilizer,
**characterized in that** the reaction takes place in the absence of any solvent.

2. The process according to claim 1, wherein **R¹** denotes a cyclohexyl group which is unsubstituted or substituted with one, two or three substituents selected from the group consisting of straight-chained or branched C1-3 alkyl and straight-chained or branched C1-3 alkyloxy, or a benzyl group.

3. The process according to claim 1 or 2, wherein the catalyst is used in an amount of 3.5 mol% to 7 mol%, based on the amount of the 2-hydroxybenzoate of general formula (III) used as starting material.

4. The process according to Claim 1, 2 or 3, wherein the stabilizer is selected from the group consisting of phenothiazine, 2,2,6,6-tetramethylpiperidinyl-N-oxyl (TEMPO), 4-hydroxy-2,2,6,6-tetramethylpiperidinyl-N-oxyl (TEMPOL), 4-(meth)acryloyloxy-2,2,6,6-tetramethylpiperidinyl-N-oxyl (TEMPOL-(meth)acrylate), hydroquinone monomethyl ether (HQME), 2,4-dimethyl-6-tert-butylphenol (DMBP), 2,6-di-tert-butylphenol, 4-methyl-2,6-di-tert-butylphenol (BHT) and mixtures thereof.

5. The process according to Claim 1, 2, 3 or 4, wherein the stabilizer is selected from the group consisting of phenothiazine, hydroquinone monomethyl ether (HQME), 2,4-dimethyl-6-tert-butylphenol (DMBP), 4-methyl-2,6-di-tert-butylphenol (BHT) and mixtures of these substances.

6. The process according to Claim 1, 2, 3, 4 or 5, wherein the stabilizer is used in amounts of 1 ppm to 5000 ppm, preferably 1 ppm to 3000 ppm, more preferably 1 ppm to 2000 ppm, based on the amount of the 2-hydroxybenzoate of general formula (III) used as starting material.

7. The process according to Claim 1, 2, 3, 4, 5 or 6, wherein the reaction time of step (a) is at least 4 hours, preferably at least 6 hours, more preferably at least 8 hours, and at most 15 hours.

8. The process according to Claim 1, 2, 3, 4, 5, 6 or 7, wherein the reaction of step (a) takes place at temperatures of 95°C to 115°C, preferably 100°C to 110°C.

9. The process according to Claim 1, 2, 3, 4, 5, 6, 7 or 8, wherein (meth)acrylic acid anhydride is used in an excess of 1.5 to 2.5 mol equivalents, preferably about 2 mol equivalents, based on the amount of the 2-hydroxybenzoate of general formula (III) used in step (a).

10. The process according to Claim 1, 2, 3, 4, 5, 6, 7, 8 or 9, wherein the 2-(meth)acryloyloxy benzoic acid ester is 3,3,5-trimethylcyclohexyl salicyl (meth)acrylate of general formula (la): 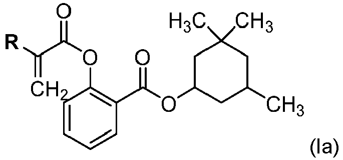 wherein R denotes H or CH₃,
comprising the step of:
(a) reacting 3,3,5-trimethylcyclohexyl-2-hydroxybenzoate of formula (Illa): 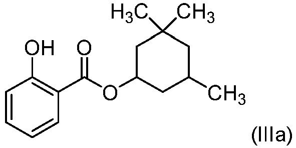
with (meth)acrylic acid anhydride of general formula (IV) 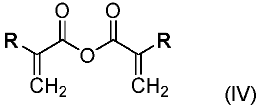
wherein R denotes H or CH₃,
in the presence of 3.5 mol% to 10 mol% of lithium methoxide as catalyst and at least one stabilizer,
**characterized in that** the reaction takes place in the absence of any solvent.

11. The process according to Claim 1, 2, 3, 4, 5, 6, 7, 8, 9 or 10, comprising the following further steps:
(b) mixing the process mixture retrieved in step (a) with water in a ratio of 1:10 to 10:1, preferably 1:1 to 10:1, and stirring the resulting mixture for at least 10 minutes;
(c) separating the aqueous phase of the mixture retrieved in step (b) from the product phase to remove the catalyst;
(d) distillation of the product phase retrieved in step (c) to retrieve the desired product with a content of (meth)acrylic acid and (meth)acrylic acid anhydride of below 1%; and
(e) optionally, subsequent additional distillation of the product retrieved under step (d) to separate it from further impurities.

12. The process according to claim 11, wherein the phase separation step (c) is carried out by using a centrifuge, separator or coalescer, preferably a centrifuge.

## Patentansprüche

1. Verfahren zur Herstellung von 2-(Meth)acryloyloxy-benzoesäureestern der allgemeinen Formel (I): 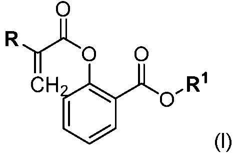 wobei
R H oder CH₃ bedeutet, und
**R¹** eine C5-7-Cycloalkylgruppe bedeutet, die unsubstituiert oder mit einem, zwei oder drei Substituenten substituiert ist, die ausgewählt sind aus der Gruppe bestehend aus geradkettigem oder verzweigtem C1-6-Alkyl, geradkettigem oder verzweigtem C1-6-Alkyloxy, -O(CO)-(C1-6)-Alkyl, -COO-(C1-6)-Alkyl, oder R¹ einen Rest der allgemeinen Formel (II) bedeutet: 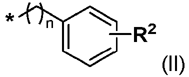
wobei
R² eine geradkettige oder verzweigte C1-6-Alkylgruppe oder eine geradkettige oder verzweigte C1-6-Alkyloxygruppe bedeutet, und
n eine ganze Zahl 0, 1 oder 2 bedeutet,
wobei das Verfahren den folgenden Schritt umfasst:
(a) Umsetzen eines 2-Hydroxybenzoats der allgemeinen Formel (III): 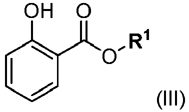 wobei
R¹ wie hierin zuvor offenbart definiert ist,
mit (Meth)acrylsäureanhydrid der allgemeinen Formel (IV): 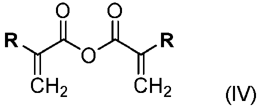
wobei R H oder CH₃ bedeutet,
in Gegenwart von Lithiummethoxid als Katalysator und mindestens einem Stabilisator,
**dadurch gekennzeichnet, dass** die Umsetzung in Abwesenheit von jeglichem Lösungsmittel erfolgt.

2. Verfahren nach Anspruch 1, wobei R¹ eine Cyclohexylgruppe, die unsubstituiert oder mit einem, zwei oder drei Substituenten substituiert ist, ausgewählt aus der Gruppe bestehend aus geradkettigem oder verzweigtem C1-3-Alkyl und geradkettigem oder verzweigtem C1-3-Alkyloxy, oder eine Benzylgruppe bedeutet.

3. Verfahren nach Anspruch 1 oder 2, wobei der Katalysator in einer Menge von 3,5 Mol-% bis 7 Mol-% verwendet wird, bezogen auf die als Ausgangsmaterial verwendete Menge des 2-Hydroxybenzoats der allgemeinen Formel (III).

4. Verfahren nach Anspruch 1, 2 oder 3, wobei der Stabilisator ausgewählt ist aus der Gruppe bestehend aus Phenothiazin, 2,2,6,6-Tetramethylpiperidinyl-N-oxyl (TEMPO), 4-Hydroxy-2,2,6,6-tetramethylpiperidinyl-N-oxyl (TEMPOL), 4-(Meth)acryloyloxy-2,2,6,6-tetramethylpiperidinyl-N-oxyl (TEMPOL-(meth)acrylat), Hydrochinonmonomethylether (HQME), 2,4-Dimethyl-6-tert.-butylphenol (DMBP), 2,6-Di-tert.-butylphenol, 4-Methyl-2,6-di-tert.-butylphenol (BHT) und Mischungen davon.

5. Verfahren nach Anspruch 1, 2, 3 oder 4, wobei der Stabilisator ausgewählt ist aus der Gruppe bestehend aus Phenothiazin, Hydrochinonmonomethylether (HQME), 2,4-Dimethyl-6-tert.-butylphenol (DMBP), 4-Methyl-2,6-di-tert.-butylphenol (BHT) und Mischungen dieser Substanzen.

6. Verfahren nach Anspruch 1, 2, 3, 4 oder 5, wobei der Stabilisator in Mengen von 1 ppm bis 5000 ppm, vorzugsweise 1 ppm bis 3000 ppm, bevorzugter 1 ppm bis 2000 ppm verwendet wird, bezogen auf die Menge des als Ausgangsmaterial verwendeten 2-Hydroxybenzoats der allgemeinen Formel (III).

7. Verfahren nach Anspruch 1, 2, 3, 4, 5 oder 6, wobei die Reaktionszeit von Schritt (a) mindestens 4 Stunden, vorzugsweise mindestens 6 Stunden, bevorzugter mindestens 8 Stunden und höchstens 15 Stunden beträgt.

8. Verfahren nach Anspruch 1, 2, 3, 4, 5, 6 oder 7, wobei die Umsetzung von Schritt (a) bei Temperaturen von 95 °C bis 115 °C, vorzugsweise 100 °C bis 110 °C erfolgt.

9. Verfahren nach Anspruch 1, 2, 3, 4, 5, 6, 7 oder 8, wobei (Meth)acrylsäureanhydrid in einem Überschuss von 1,5 bis 2,5 Moläquivalenten, vorzugsweise etwa 2 Moläquivalenten verwendet wird, bezogen auf die Menge des in Schritt (a) verwendeten 2-Hydroxybenzoats der allgemeinen Formel (III).

10. Verfahren nach Anspruch 1, 2, 3, 4, 5, 6, 7, 8 oder 9, wobei der 2-(Meth)acryloyloxybenzoesäureester 3,3,5-Trimethylcyclohexylsalicyl(meth)acrylat der allgemeinen Formel (Ia) ist: 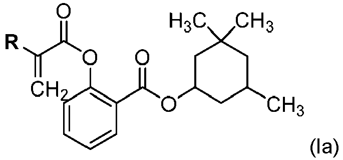
wobei R H oder CH₃ bedeutet,
welches den folgenden Schritt umfasst:
(a) Umsetzen von 3,3,5-Trimethylcyclohexyl-2-hydroxybenzoat der Formel (IIIa): 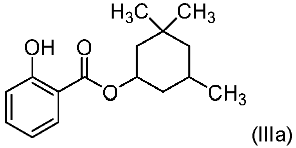
mit (Meth)acrylsäureanhydrid der allgemeinen Formel (IV): 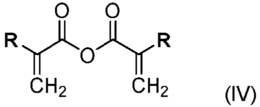
wobei R H oder CH₃ bedeutet,
in Gegenwart von 3,5 Mol-% bis 10 Mol-% Lithiummethoxid als Katalysator und mindestens einem Stabilisator,
**dadurch gekennzeichnet, dass** die Umsetzung in Abwesenheit von jeglichem Lösungsmittel erfolgt.

11. Verfahren nach Anspruch 1, 2, 3, 4, 5, 6, 7, 8, 9 oder 10, umfassend die folgenden weiteren Schritte:
(b) Mischen der in Schritt (a) gewonnenen Verfahrensmischung mit Wasser im Verhältnis 1:10 bis 10:1, vorzugsweise 1:1 bis 10:1, und Rühren der erhaltenen Mischung für mindestens 10 Minuten;
(c) Trennen der wässrigen Phase der in Schritt (b) gewonnenen Mischung von der Produktphase, um den Katalysator zu entfernen;
(d) Destillieren der in Schritt (c) gewonnenen Produktphase, um das gewünschte Produkt mit einem Gehalt an (Meth)acrylsäure und (Meth)acrylsäureanhydrid von unter 1 % zu gewinnen; und
(e) gegebenenfalls anschließendes zusätzliches Destillieren des in Schritt (d) gewonnenen Produkts, um es von weiteren Verunreinigungen zu trennen.

12. Verfahren nach Anspruch 11, wobei der Phasentrennschritt (c) unter Verwendung einer Zentrifuge, eines Separators oder eines Koaleszers, vorzugsweise einer Zentrifuge, durchgeführt wird.

## Revendications

1. Procédé de préparation d'esters d'acide 2-(méth)acryloyloxybenzoïque de formule générale (I) 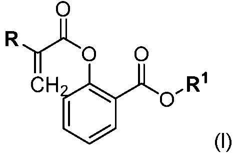 où
**R** représente H ou CH₃ et
**R¹** représente un groupe cycloalkyle en C5-7 qui est non substitué ou substitué par un, deux ou trois substituants choisis dans le groupe constitué par alkyle en C1-6 à chaîne droite ou ramifié, alcoxy en C1-6 à chaîne droite ou ramifié, -O(CO)-(alkyle en C1-6), -COO-(alkyle en C1-6) ou
**R¹** représente un résidu de formule générale (II) 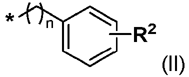
où
**R²** représente un groupe alkyle en C1-6 à chaîne droite ou ramifié ou un groupe alcoxy en C1-6 à chaîne droite ou ramifié et
**n** représente un nombre entier 0, 1 ou 2,
le procédé comprenant l'étape consistant à :
(a) faire réagir un 2-hydroxybenzoate de formule générale (III) : 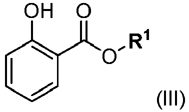
où
**R¹** est défini comme divulgué ci-dessus,
avec de l'anhydride d'acide (méth)acrylique de formule générale (IV) 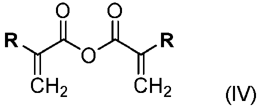
où **R** représente H ou CH₃,
en présence de méthylate de lithium en tant que catalyseur et d'au moins un stabilisant,
**caractérisé en ce que** la réaction a lieu en l'absence d'un quelconque solvant.

2. Procédé selon la revendication 1, dans lequel **R¹** représente un groupe cyclohexyle, qui est non substitué ou substitué par un, deux ou trois substituants choisis dans le groupe constitué par alkyle en C1-3 à chaîne droite ou ramifié et alcoxy en C1-3 à chaîne droite ou ramifié, ou un groupe benzyle.

3. Procédé selon la revendication 1 ou 2, dans lequel le catalyseur est utilisé en une quantité de 3,5 % en mole à 7 % en mole, par rapport à la quantité du 2-hydroxybenzoate de formule générale (III) utilisé en tant que matière de départ.

4. Procédé selon la revendication 1, 2 ou 3, dans lequel le stabilisant est choisi dans le groupe constitué par la phénothiazine, le 2,2,6,6-tétraméthylpipéridinyl-N-oxyle (TEMPO), le 4-hydroxy-2,2,6,6-tétraméthylpipéridinyl-N-oxyle (TEMPOL), le 4-(méth)acryloyloxy-2,2,6,6-tétraméthylpipéridinyl-N-oxyle (TEMPOL-(méth)acrylate), l'éther monométhylique d'hydroquinone (HQME), le 2,4-diméthyl-6-tert-butylphénol (DMBP), le 2,6-di-tert-butylphénol, le 4-méthyl-2,6-di-tert-butylphénol (BHT) et les mélanges de ceux-ci.

5. Procédé selon la revendication 1, 2, 3 ou 4, dans lequel le stabilisant est choisi dans le groupe constitué par la phénothiazine, l'éther monométhylique d'hydroquinone (HQME), le 2,4-diméthyl-6-tert-butylphénol (DMBP), le 4-méthyl-2,6-di-tert-butylphénol (BHT) et les mélanges de ces substances.

6. Procédé selon la revendication 1, 2, 3, 4 ou 5, dans lequel le stabilisant est utilisé en quantités de 1 ppm à 5000 ppm, de préférence de 1 ppm à 3000 ppm, plus préférablement de 1 ppm à 2000 ppm, par rapport à la quantité du 2-hydroxybenzoate de formule générale (III) utilisé en tant que matière de départ.

7. Procédé selon la revendication 1, 2, 3, 4, 5 ou 6, dans lequel la durée de réaction de l'étape (a) est d'au moins 4 heures, de préférence d'au moins 6 heures, plus préférablement d'au moins 8 heures et d'au maximum 15 heures.

8. Procédé selon la revendication 1, 2, 3, 4, 5, 6 ou 7, dans lequel la réaction de l'étape (a) a lieu à des températures de 95 °C à 115 °C, de préférence de 100 °C à 110 °C.

9. Procédé selon la revendication 1, 2, 3, 4, 5, 6, 7 ou 8, dans lequel l'anhydride d'acide (méth)acrylique est utilisé en un excès de 1,5 à 2,5 équivalents molaires, de préférence d'environ 2 équivalents molaires, par rapport à la quantité du 2-hydroxybenzoate de formule générale (III) utilisé à l'étape (a).

10. Procédé selon la revendication 1, 2, 3, 4, 5, 6, 7, 8 ou 9, dans lequel l'ester d'acide 2-(méth)acryloyloxybenzoïque est le (méth)acrylate de 3,3,5-triméthylcyclohexylsalicyle de formule générale (Ia) : 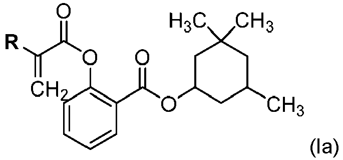
où R représente H ou CH₃,
comprenant l'étape consistant à :
(a) faire réagir du 2-hydroxybenzoate de 3,3,5-triméthylcyclohexyle de formule (IIIa) : 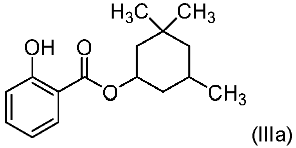
avec de l'anhydride d'acide (méth)acrylique de formule générale (IV) 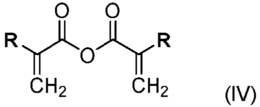
où **R** représente H ou CH₃,
en présence de 3,5 % en mole à 10 % en mole de méthylate de lithium en tant que catalyseur et d'au moins un stabilisant,
**caractérisé en ce que** la réaction a lieu en l'absence d'un quelconque solvant.

11. Procédé selon la revendication 1, 2, 3, 4, 5, 6, 7, 8, 9 ou 10 comprenant les autres étapes suivantes :
(b) le mélange du mélange de procédé récupéré à l'étape (a) avec de l'eau en un rapport de 1:10 à 10:1, de préférence de 1:1 à 10:1, et l'agitation du mélange résultant pendant au moins 10 minutes ;
(c) la séparation de la phase aqueuse du mélange récupéré à l'étape (b) de la phase de produit pour éliminer le catalyseur ;
(d) la distillation de la phase de produit récupérée à l'étape (c) pour récupérer le produit souhaité avec une teneur en acide (méth)acrylique et en anhydride d'acide (méth)acrylique au-dessous de 1 % ; et
(e) éventuellement, la distillation supplémentaire ultérieure du produit récupéré dans l'étape (d) pour le séparer d'autres impuretés.

12. Procédé selon la revendication 11, dans lequel l'étape de séparation de phases (c) est effectuée à l'aide d'une centrifugeuse, d'un séparateur ou d'un coalesceur, de préférence d'une centrifugeuse.
